# EUROPEAN PATENT APPLICATION

(11) **EP 4 467 983 A1**
(43) Date of publication of application: **27.11.2024**
(21) Application number: 23175751.9
(22) Date of filing: 26.05.2023
(51) Int. Cl.: G01N 33/566

(54) **MOLECULAR IDENTIFICATION OF COMPOUNDS TARGETING TRISTETRAPROLIN (TTP) AND TTP PHOSPHORYLATION**

(71) Applicant: King Faisal Specialist Hospital & Research Centre, Riyadh, 11211 (SA)
(72) Inventor: KHALID S., Abu Khabar, Riyadh 11211 (SA)
(74) Representative: Engelhard, Markus

(57) **Abstract**

The present invention relates to a method of identifying, optionally using molecular docking and/or in vitro assays, a compound which binds to tristetraprolin (TTP), preferably a compound which inhibits phosphorylation of TTP. Furthermore, the present invention relates to a method of preventing or treating a disease in a patient having an increased level of phosphorylated TTP. The present invention also relates to a compound for use in a method of preventing or treating a disease in a patient having an increased level of phosphorylated TTP.

## Description

### FIELD OF THE INVENTION

The present invention relates to a method of identifying a compound which binds to tristetraprolin (TTP), preferably a compound which inhibits phosphorylation of TTP. Furthermore, the present invention relates to a method of preventing or treating a disease in a patient having an increased level of phosphorylated TTP. The present invention also relates to a compound for use in a method of preventing or treating a disease in a patient having an increased level of phosphorylated TTP.

### BACKGROUND OF THE INVENTION

The regulation of mRNA stability is an important control mechanism for gene expression. Loss of mRNA stability regulation may result in diseases where there is overexpression of genes encoding proto-oncogenes, growth factors, inflammatory cytokines, and other genes involved in disease development. Important determinants of mRNA stability and regulation are adenylate/uridylate(AU)-rich elements (AREs).

A key RNA-binding protein that promotes AU-rich mRNA deadenylation and decay is the zinc finger protein tristetraprolin (TTP). TTP is a destabilizing mRNA binding protein which regulates gene expression of a variety of targets, including targets involved in inflammation and cancer. TTP expression, regulation and activity is controlled by TTP phosphorylation.

Many human tumors are found to be associated with deficiency of TTP. For example, deficiency of TTP is linked to hallmarks of cancer. The aberrant expression or activity of TTP is associated with changes at different levels of regulation, including transcriptional (e.g. epigenetic), post-transcriptional, and post-translational regulation. Phosphorylation of TTP by various protein kinases is one of the posttranslational modifications that profoundly affect its cellular localization and activity [1], [2], [3]. For example, the p38/MK2 is involved in TTP phosphorylation and is involved in preventing its ability to recruit the mRNA decay machinery which leads to overproduction of ARE-mRNA products. Previous reports indicate that phosphorylation events during inflammation lead to stabilization of TTP and that dephosphorylated TTP is unstable and less abundant in cells [1],[2]. Unlike the active unphosphorylated TTP, MK2-phosphorylated TTP is of increased abundance due to protein stabilization, and is less active.

It has been shown that TTP has multiple phosphorylation sites, and thus can be affected by several signaling pathways and many kinases [4]. For example, major MK2 sites for TTP phosphorylation are mouse/human serine 52/60 and 178/186. However, there are many other potential amino acid sites for phosphorylation and for a variety of kinase targets which may play a role in TTP-related diseases. TTP, particularly TTP phosphorylation, may play an important role in various diseases including cancer.

Due to the importance of TTP in disease development, there is a need to identify compounds that bind to and/or inhibit TTP. Particularly, there is the need to identify compounds that inhibit TTP phosphorylation. There is also a need for efficient methods of identifying compounds binding to and/or inhibiting TTP, such as inhibiting TTP phosphorylation. Moreover, there is the need for efficient methods of screening compound libraries for compounds binding to TTP. Furthermore, there remains the need for effective methods of preventing or treating diseases which are associated with aberrant TTP, such as diseases which are associated with increased levels of TTP phosphorylation. There also remains the need to provide compounds for use in preventing or treating diseases which are associated with aberrant TTP, particularly aberrant TTP phosphorylation.

### SUMMARY OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In a first aspect, the present invention relates to a method of identifying a compound which binds to tristetraprolin (TTP), preferably a compound which inhibits phosphorylation of TTP, comprising the following steps:
a) providing a three-dimensional structure of TTP; wherein, preferably, said providing comprises predicting a three-dimensional structure of TTP using a computer-implemented method;
b) preferably, identifying a phosphorylation site of TTP; wherein, preferably, said phosphorylation site of TTP comprises or consists of serine and/or threonine, preferably serine;
c) identifying a compound which binds to said three-dimensional structure of TTP provided in step a), preferably a compound which binds to said phosphorylation site of TTP identified in step b), if present; wherein, optionally, said identifying comprises virtually screening a compound library comprising at least one compound;
d) optionally, determining whether said compound identified in step c) inhibits phosphorylation of TTP, preferably using an in vitro assay, optionally a cell bioassay.

In one embodiment, said providing in step a) comprises predicting a three-dimensional structure of TTP using a computer-implemented method comprising fold recognition; wherein, preferably, said predicting comprises secondary structure prediction, template detection, fragment structure assembly, model selection, atomic-level structure refinement, and/or structure-based biology function annotation;
wherein, more preferably, said predicting comprises iterative threading assembly refinement.

In one embodiment, said method comprises identifying a phosphorylation site of TTP, wherein said identifying a phosphorylation site comprises predicting a phosphorylation site using a computer-implemented method and/or comprises site-directed mutagenesis.

In one embodiment, said identifying a compound in step c) comprises identifying a compound which binds to said three-dimensional structure of TTP provided in step a), preferably a compound which binds to said phosphorylation site of TTP identified in step b), if present; wherein said identifying comprises screening, preferably virtually screening, a compound library comprising at least one compound, preferably a plurality of compounds; wherein, optionally, said at least one compound, preferably said plurality of compounds, is/are selected from small molecule inhibitors, antibodies, and antigen-binding fragments thereof, preferably from protein kinase inhibitors.

In one embodiment, said identifying a compound in step c) comprises virtually screening a compound library comprising at least one compound by molecular docking of said at least one compound to TTP, preferably comprising determining whether said at least one compound binds to TTP;
wherein, preferably, said identifying a compound in step c) comprises virtually screening a compound library comprising at least one compound by molecular docking of said at least one compound to said phosphorylation site of TTP identified in step b), if present, preferably comprising determining whether said at least one compound binds to TTP, more preferably comprising determining whether said at least one compound inhibits phosphorylation of TTP.

In one embodiment, said identifying a compound in step c) comprises virtually screening a compound library comprising at least one compound by molecular docking of said at least one compound to TTP, preferably to said phosphorylation site of TTP identified in step b), if present, wherein said virtually screening comprises docking of said at least one compound to a set of grids describing the three-dimensional structure of TTP provided in step a), preferably describing said phosphorylation site of TTP identified in step b), if present.

In one embodiment, said TTP in step a) is human TTP, preferably TTP having an amino acid sequence of SEQ ID NO. 1.

In one embodiment, said method, optionally said step c), comprises determining whether said compound binds to residues S60, S102, T106, S186, and/or S323 of TTP, preferably to residues S60, S186, and/or S323, wherein TTP has an amino acid sequence of SEQ ID NO. 1.

In one embodiment, the compound identified in step c) binds to said TTP with a binding free energy of less than -5 kcal/mol, preferably less than -7 kcal/mol, more preferably less than -8 kcal/mol.

In one embodiment, said identifying in step c) comprises virtually screening a compound library comprising at least 5 compounds, preferably at least 10 compounds, more preferably at least 100 compounds, even more preferably at least 500 compounds, even more preferably at least 1000 compounds such as at least 1400 compounds; wherein, optionally, said compounds are selected from small molecule inhibitors, antibodies, and antigen-binding fragments thereof, preferably from protein kinase inhibitors;
wherein, optionally, said identifying comprises identifying one or more compounds binding to said TTP with a binding free energy of less than -7 kcal/mol, preferably less than -8 kcal/mol, and/or identifying one or more compounds having a lower binding free energy than 80%, preferably than 90% of the compounds of the compound library.

In one embodiment, said method comprises step d) of determining whether said compound identified in step c) inhibits phosphorylation of TTP, wherein said determining comprises:
i) providing a sample comprising cells, optionally a tissue sample;
ii) treating said sample comprising cells, preferably immune cells and/or tumor cells, more preferably monocytes and/or macrophages e.g. THP-1 cells, with said compound identified in step c);
iii) optionally, stimulating said cells with a TTP phosphorylation stimulating agent, preferably with a lipopolysaccharide, IL-1, and/or PMA;
iv) determining whether said compound reduces a level of phosphorylated TTP in said treated sample compared to a control, wherein said control is a level of phosphorylated TTP determined in said sample prior to said treating in step ii), and/or is a reference value, and/or is a level of phosphorylated TTP determined in a reference sample;
wherein a reduction in the level of phosphorylated TTP indicates that said compound inhibits phosphorylation of TTP.

In one embodiment, said reduction is a reduction by at least 15 %, preferably by at least 20 %, more preferably by at least 25 %; and/or
said level of phosphorylated TTP is determined using an antibody or antigen-binding fragment thereof targeting phosphorylated TTP.

In one embodiment, said method comprises step d) of determining whether said compound identified in step c) inhibits phosphorylation of TTP, wherein said determining comprises Western blotting, immunofluorescence, immunohistochemistry, immunocytochemistry, and/or ELISA.

In a further aspect, the present invention relates to a method of preventing or treating a disease in a patient having an increased level of phosphorylated TTP; wherein, preferably, said disease is selected from cancer, inflammatory diseases, autoimmune diseases, and aging-associated diseases; wherein said method comprises administering a therapeutically effective amount of a compound identified using a method of identifying a compound which binds to tristetraprolin (TTP), as defined herein, to a patient in need thereof.

In one embodiment, the compound is selected from acetyldigitoxin, digitoxin, ergotamine, bromocriptine, dihydroergotoxine, dihydroergotamine, ergoloid mesylate, conivaptan, teniposide, tubocurarin, differin, dutasteride, itraconazole, paclitaxel, lurasidone, novobiocin, praziquantel, loperamide, pranlukast, fluspirilene, antrafenine, desloratadin, dihydroergotamine, ergotamine, glimepiride, glipizide, darifenacin, tadalafil, conivaptan, danazol, dutasteride, tasosartan, metocurine, nilotinib, telithromycin, irinotecan, 5-bromo-2-chloro-N-[3-methyl-4-(2-oxo-2H-chromen-3-yl)phenyl]benzamide, maraviroc, and saquinavir;
wherein, preferably, the compound is selected from glipizide, loperamide, praziquantel, glimepiride, fluspirilene, desloratadin, ergotamine, and teniposide.

In a further aspect, the present invention relates to a compound for use in a method of preventing or treating a disease in a patient having an increased level of phosphorylated TTP; wherein, preferably, said disease is selected from cancer, inflammatory diseases, autoimmune diseases, and aging-associated diseases; wherein said method comprises administering a therapeutically effective amount of a compound identified using a method of identifying a compound which binds to tristetraprolin (TTP), as defined herein, to a patient in need thereof.

In one embodiment, the compound is selected from acetyldigitoxin, digitoxin, ergotamine, bromocriptine, dihydroergotoxine, dihydroergotamine, ergoloid mesylate, conivaptan, teniposide, tubocurarin, differin, dutasteride, itraconazole, paclitaxel, lurasidone, novobiocin, praziquantel, loperamide, pranlukast, fluspirilene, antrafenine, desloratadin, dihydroergotamine, ergotamine, glimepiride, glipizide, darifenacin, tadalafil, conivaptan, danazol, dutasteride, tasosartan, metocurine, nilotinib, telithromycin, irinotecan, 5-bromo-2-chloro-N-[3-methyl-4-(2-oxo-2H-chromen-3-yl)phenyl]benzamide, maraviroc, and saquinavir;
wherein, preferably, the compound is selected from glipizide, loperamide, praziquantel, glimepiride, fluspirilene, desloratadin, ergotamine, and teniposide.

In one embodiment, said method of preventing or treating a disease in a patient having an increased level of phosphorylated TTP is as defined herein.

In a further aspect, the present invention relates to a use of a compound for the manufacture of a medicament for preventing or treating a disease in a patient having an increased level of phosphorylated TTP; wherein, preferably, said disease is selected from cancer, inflammatory diseases, autoimmune diseases, and aging-associated diseases; wherein said compound is a compound identified using a method of identifying a compound which binds to tristetraprolin (TTP), as defined herein.

In one embodiment, said compound and/or said preventing or treating a disease in a patient having an increased level of phosphorylated TTP is/are as defined herein.

### DETAILED DESCRIPTION

The present invention aims at identifying compounds which bind to TTP. Furthermore, the present invention aims at providing an efficient method of screening a compound library comprising a plurality of compounds to identify compounds that bind to TTP, particularly that bind to TTP with a high affinity. Moreover, in view of the importance of phosphorylated TTP as a biomarker, such as a biomarker for cancer, the present invention aims at providing an efficient method of identifying compounds which bind to the phosphorylation site of TTP.

Furthermore, the present invention aims at providing a method for identifying effective therapeutic compounds, such as compounds that maybe used in the treatment of diseases such as cancer, inflammatory diseases, autoimmune diseases, or aging-associated diseases.

The present invention relates to a method of identifying a compound which binds to tristetraprolin (TTP), preferably a compound which inhibits phosphorylation of TTP, comprising the following steps:
a) providing a three-dimensional structure of TTP; wherein, preferably, said providing comprises predicting a three-dimensional structure of TTP using a computer-implemented method;
b) preferably, identifying a phosphorylation site of TTP; wherein, preferably, said phosphorylation site of TTP comprises or consists of serine and/or threonine, preferably serine;
c) identifying a compound which binds to said three-dimensional structure of TTP provided in step a), preferably a compound which binds to said phosphorylation site of TTP identified in step b), if present; wherein, optionally, said identifying comprises virtually screening a compound library comprising at least one compound;
d) optionally, determining whether said compound identified in step c) inhibits phosphorylation of TTP, preferably using an in vitro assay, optionally a cell bioassay.

Advantageously, the method of identifying a compound according to the invention allows to identify compounds which effectively bind to TTP and which may be used in the treatment of diseases associated with TTP and/or phosphorylated TTP, such as cancer, inflammatory diseases, autoimmune diseases, and/or aging-associated diseases. Advantageously, the method of the invention uses the three-dimensional structure of TTP to identify compounds which efficiently bind to TTP. In one embodiment, the identified compound target(s) phosphorylated TTP or unphosphorylated TTP, i.e. specifically binds to phosphorylated TTP or unphosphorylated TTP.

In one embodiment, said TTP is human TTP, preferably TTP having an amino acid sequence of SEQ ID NO. 1. In one embodiment, said binding to TTP comprises or consists of binding to a phosphorylation site of TTP. In one embodiment, said binding to TTP comprises or consists of a specific binding to TTP, preferably a specific binding to a phosphorylation site of TTP. For example, said binding may relate to a binding of said compound to an amino acid at a position of an amino acid sequence of TTP, preferably an amino acid sequence of SEQ ID NO. 1, particularly selected from amino acids at positions 60, 102, 106, 186, and 323 of TTP. In one embodiment, said TTP is human TTP; preferably TTP having an amino acid sequence of SEQ ID NO. 1 which has the following amino acid sequence:

MDLTAIYESLLSLSPDVPVPSDHGGTESSPGWGSSGPWSLSPSDSSPSGVTSRLPGRSTSLVE
GRSCGWVPPPPGFAPLAPRLGPELSPSPTSPTATSTTPSRYKTELCRTFSESGRCRYGAKCQFA
HGLGELRQANRHPKYKTELCHKFYLQGRCPYGSRCHFIHNPSEDLAAPGHPPVLRQSISFSG
LPSGRRTSPPPPGLAGPSLSSSSFSPSSSPPPPGDLPLSPSAFSAAPGTPLARRDPTPVCCPSCR
RATPISVWGPLGGLVRTPSVQSLGSDPDEYASSGSSLGGSDSPVFEAGVFAPPQPVAAPRRLPI
FNRISVSE.

The terms "three-dimensional structure of TTP" and "tertiary structure of TTP", as used herein, may be used interchangeably. In one embodiment, said step a) of providing a three-dimensional structure of TTP comprises predicting said structure, preferably using computational biology. The three-dimensional structure of TTP may be predicted with any method known to the person skilled in the art. In one embodiment, said providing a three-dimensional structure of TTP comprises homology modeling and/or fold recognition. In a preferred embodiment, said providing comprises predicting a three-dimensional structure of TTP using a computer-implemented method. For example, the three-dimensional structure may be predicted using I-TASSER, Autodock, vina, Glide, HHpred, and/or AlphaFold, preferably using I-TASSER such as I-TASSER version 5.2. In one embodiment, said providing, preferably predicting the three-dimensional structure comprises using a computer-implemented method involving artificial intelligence.

In one embodiment, said providing in step a) comprises predicting a three-dimensional structure of TTP using an amino acid sequence of TTP, optionally using an amino acid sequence of TTP having SEQ ID No.1. In one embodiment, said providing in step a) comprises predicting a three-dimensional structure of TTP using a computer-implemented method comprising fold recognition. In one embodiment, said predicting of the three-dimensional structure comprises reassembling structural fragments from threading templates, e.g. using Monte Carlo simulations such as replica exchange Monte Carlo simulations. In one embodiment, the terms "protein threading" and "fold recognition" are used interchangeably. In one embodiment, the term "fold recognition", as used herein, preferably relates to a method of protein modeling which is used to model those proteins which have the same fold as proteins of known structures, but do not have homologous proteins with known structure, such as to model TTP. In one embodiment, said predicting comprises aligning each amino acid in the target sequence, for example SEQ ID NO.1, to a position in a template structure.

In one embodiment, said predicting comprises selecting protein structures from protein structure databases as structural templates; designing a suitable scoring function to measure the fitness between target sequences and templates based on the knowledge of the known relationships between the structures and the sequences; aligning the target sequence, particularly a sequence of TTP, with each of the structure templates by optimizing the designed scoring function; selecting a threading alignment that is statistically most probable as the threading prediction; constructing a structure model for the target, particularly the three-dimensional structure of TTP, by placing the backbone atoms of the target sequence at their aligned backbone positions of the selected structural template.

In one embodiment, said predicting comprises secondary structure prediction, template detection, fragment structure assembly, model selection, atomic-level structure refinement, and/or structure-based biology function annotation. In one embodiment, said predicting comprises iterative threading assembly refinement. In one embodiment, said predicting comprises secondary structure prediction, template detection, fragment structure assembly, model selection, atomic-level structure refinement, and/or structure-based biology function annotation, optionally comprises iterative threading assembly refinement, for example using I-TASSER. For example, said secondary structure prediction may be performed using PSSpred. For example, said template detection may be performed using LOMETS (particularly a set of locally installed threading programs for meta-server protein fold-recognition). For example, said fragment structure assembly may be performed using replica-exchange Monte Carlo simulation. For example, said model selection maybe performed by clustering structure decoys using SPICKER (particularly a clustering program to identify near-native protein model from structure decoys). For example, said atomic-level structure refinement may be performed by fragment-guided molecular dynamics simulation or ModRefiner. For example, said structure-based biology function annotation may be performed using COACH (particularly a function annotation program based on COFACTOR, TM-SITE and S-SITE).

In one embodiment, said method comprises step b) of identifying a phosphorylation site of TTP, particularly one or more phosphorylation sites of TTP. For example, the phosphorylation site may be a target site of a kinase phosphorylating said TTP. In one embodiment, one or more phosphorylation sites of TTP are identified, particularly identified in an amino acid sequence of TTP, in a secondary structure of TTP, and/or in a tertiary structure of TTP. In one embodiment, said phosphorylation site of TTP comprises or consists of serine and/or threonine, preferably serine. In one embodiment, said identifying one or more phosphorylation sites comprises computational phosphorylation site prediction. In one embodiment, said method comprises identifying a phosphorylation site of TTP, wherein said identifying a phosphorylation site comprises predicting a phosphorylation site using a computer-implemented method, particularly computational phosphorylation site prediction, and/or comprises site-directed mutagenesis. For example, said identifying a phosphorylation site may comprise predicting a phosphorylation site and subsequently further analyzing the predicted phosphorylation sites by site-directed mutagenesis. In one embodiment, said identifying a phosphorylation site of TTP, optionally said predicting a phosphorylation site, comprises using PhospoSite^{®}.

In one embodiment, said identifying a compound in step c) comprises identifying a compound which binds to said three-dimensional structure of TTP provided in step a), preferably a compound which binds to said phosphorylation site of TTP identified in step b), if present; wherein said identifying comprises screening, preferably virtually screening, a compound library comprising at least one compound, preferably a compound library comprising a plurality of compounds. For example, said screening may be performed using an in vitro screening, such as a high-throughput in vitro screening, and/or using virtually screening. For example, said identifying a compound in step c) may comprise virtually screening a compound library comprising a plurality of compounds, and subsequently screening a subset of compounds identified using said virtually screening in an in vitro screening, such as a high-throughput in vitro screening. Advantageously, by virtually screening a compound library, the most promising compounds may be efficiently identified. Furthermore, advantageously, the best compounds may be efficiently identified with a subsequent in vitro screening. In one embodiment, said compound library comprises small molecule inhibitors, antibodies, and/or antigen-binding fragments thereof, preferably comprises protein kinase inhibitors.

In one embodiment, said step c) of identifying a compound which binds to the three-dimensional structure of TTP, preferably identifying a compound which binds to said phosphorylation site of TTP, comprises providing one or more, preferably a plurality of compounds to be tested. In one embodiment, said step c) of identifying a compound which binds to the three-dimensional structure of TTP, preferably identifying a compound which binds to said phosphorylation site of TTP, comprises screening, preferably virtually screening a compound library comprising at least one compound, preferably comprising a plurality of compounds. In one embodiment, said screening, preferably virtually screening a compound library comprises analyzing an affinity of the tested compound(s) to TTP. In one embodiment, said screening, preferably virtually screening a compound library comprises analyzing a binding free energy of the tested compound(s) to TTP. Advantageously, by screening a compound library comprising a plurality of compounds, those compounds of the plurality of compounds can be identified which have the highest affinity to TTP, particularly the highest affinity to a phosphorylation site of TTP. Typically, the lower the binding free energy, the higher the affinity of the compound to TTP. Advantageously, said screening of a compound library allows for a high throughput analysis of various compounds.

In one embodiment, said identifying a compound in step c) comprises providing one or more compound(s) to be tested; wherein, optionally, said providing comprises virtually providing one or more compounds to be tested and/or physically providing one or more compounds to be tested. In one embodiment, said identifying a compound in step c) comprises providing a compound library comprising one or more compound(s) to be tested. In one embodiment, said providing one or more compound(s) to be tested comprises providing one or more compound(s) suspected of binding to TTP, preferably binding to a phosphorylation site of TIP. In one embodiment, said identifying a compound in step c) comprises providing a plurality of compound(s) to be tested, preferably a compound library comprising a plurality of compounds, such as a compound library comprising at least 5 compounds, preferably at least 10 compounds, more preferably at least 100 compounds, even more preferably at least 500 compounds, even more preferably at least 1000 compounds such as at least 1400 compounds. In one embodiment, the compounds to be tested are selected from small molecule inhibitors, antibodies, and antigen-binding fragments thereof, preferably from protein kinase inhibitors. In one embodiment, the expression "to be tested" relates to being tested with respect to TTP binding characteristics, particularly affinity to TTP, optionally with respect to TTP phosphorylation reducing activity. For example, compounds to be tested may include acetyldigitoxin, digitoxin, ergotamine, bromocriptine, dihydroergotoxine, dihydroergotamine, ergoloid mesylate, conivaptan, teniposide, tubocurarin, differin, dutasteride, itraconazole, paclitaxel, lurasidone, novobiocin, praziquantel, loperamide, pranlukast, fluspirilene, antrafenine, desloratadin, dihydroergotamine, ergotamine, glimepiride, glipizide, darifenacin, tadalafil, conivaptan, danazol, dutasteride, tasosartan, metocurine, nilotinib, telithromycin, irinotecan, 5-bromo-2-chloro-N-[3-methyl-4-(2-oxo-2H-chromen-3-yl)phenyl]benzamide, maraviroc, and/or saquinavir. In one embodiment, the one or more compound(s) to be tested are selected from small molecule inhibitors, antibodies, and antigen-binding fragments thereof; preferably from protein kinase inhibitors.

The term "small molecule inhibitor" relates to a small molecule compound, preferably to a small molecule which inhibits a signaling pathway in a patient's body, such as a cancer-related signaling pathway or a signaling pathway related to an aging-associated disease. The term "antigen-binding fragment thereof, as used herein, relates to a peptide that specifically binds to an antigen. In one embodiment, an antigen-binding fragment is based on an immunoglobulin, such as a polyclonal or monoclonal antibody, for example a substantially intact antibody, a Fab fragment, a F(ab')2 fragment, a diabody, a single chain Fv fragment, a tetrabody, a triabody, a disulfide bond-stabilized Fv (dsFv), or a heavy chain VHH fragment from camels, or is based on a protein scaffold structure having antigen-binding capacity, such as an anticalin protein, an Affilin, an Affimer, an Affitin, an Alphabody, a nanobody, or a DARPin, preferably comprising antigen-binding determinants, such as a CDR, of an antibody.

The term "protein kinase inhibitor", as used herein, refers to an inhibitor that blocks the action of one or more protein kinases. In one embodiment, said term relates to an inhibitor that attenuates the action of one or more protein kinases. In one embodiment, said protein kinase inhibitor is a serine/threonine protein kinase inhibitor, such as a B-Raf kinase inhibitor or a polo-like kinase inhibitor, or a tyrosine kinase inhibitor, for example a VEGFR2 inhibitor. The term "PLK-1" or "polo-like kinase 1", as used herein, refers to a specific kinase which is a member of the family of polo-like kinases. In one embodiment, a protein kinase inhibitor is preferably an inhibitor of a MAP kinase, such as an inhibitor of MK2 and/or ERK, an inhibitor of AKT, and/or an inhibitor of ERBB2.

In one embodiment, said identifying a compound in step c) comprises virtually screening a compound library comprising at least one compound, preferably a plurality of compounds, by molecular docking of said at least one compound to TTP. In one embodiment, said identifying a compound in step c) comprises determining whether said at least one compound binds to TTP, preferably comprises determining an affinity of a compound to be tested to said TTP, e.g. by determining a binding free energy. In one embodiment, said identifying a compound in step c) comprises virtually screening a compound library comprising at least one compound, preferably by molecular docking of said at least one compound to said phosphorylation site of TTP. Virtually screening preferably comprises or consists of a computational technique; particularly a computational technique to search a compound library in order to identify those compounds which are most likely to bind to TTP, preferably a phosphorylation site of TTP. Advantageously, virtually screening allows to automatically evaluate very large libraries of compounds using a computer program. In one embodiment, said molecular docking predicts an affinity of a compound to TTP, preferably of a compound to a phosphorylation site of TTP, e.g. by predicting a binding free energy. In one embodiment, said virtually screening and/or said molecular docking comprises determining an affinity of a compound to TTP, preferably of a compound to a phosphorylation site of TTP, e.g. by determining a binding free energy. Advantageously, by virtually screening a compound library, for example involving molecular docking, TTP binding compounds are efficiently identified.

In one embodiment, said identifying a compound in step c) comprises virtually screening a compound library comprising at least one compound, preferably by molecular docking of said at least one compound to TTP, and predicting an of said at least one compound to said TTP, preferably to a phosphorylation site of TTP, e.g. by predicting a binding free energy. In one embodiment, said virtually screening comprises docking of said at least one compound to a set of grids describing the three-dimensional structure of TTP, preferably describing a phosphorylation site of TTP; wherein, preferably, said docking comprises predicting an affinity of said compound to TTP, preferably the phosphorylation site of TTP, e.g. by predicting a binding free energy. In one embodiment, at least one grid comprises a phosphorylation site of TTP, such as a serine. In one embodiment, an identified compound binds to said TTP with a binding free energy of -7 kcal/mol or less. In one embodiment, said virtually screening is performed using PyRx, optionally comprising using Auto Dock and/or Auto Dock Vina.

In one embodiment, said identifying in step c) comprises virtually screening a compound library comprising at least 5 compounds, preferably at least 10 compounds, more preferably at least 100 compounds, even more preferably at least 500 compounds, even more preferably at least 1000 compounds such as at least 1400 compounds. For example, the compound library may comprise small molecule inhibitors, antibodies, and/or antigen-binding fragments thereof, such as protein kinase inhibitors. Advantageously, using a method of the invention, large compound libraries, such as compound libraries comprising more than 1000 compounds such as at least 1400 compounds, may be analyzed in the most suitable compounds can be identified. In one embodiment, said identifying in step c) comprises identifying one or more compounds having a lower binding energy than 80%, preferably than 90% of the compounds of the compound library. In one embodiment, said identifying in step c) comprises identifying one or more compounds having a higher affinity to TTP than 80%, preferably than 90% of the compounds of the compound library. Typically, a higher affinity is indicated by a lower binding free energy. Advantageously, by identifying compound(s) having a lower binding free energy than 80%, preferably than 90% of the tested compounds, the compound(s) with the best binding characteristics may be identified.

The terms "affinity" and "binding affinity", as used herein, preferably relate to a strength of the binding interaction between a compound and TTP. For example, high-affinity binding of a tested compound to TTP may result from greater attractive forces between the compound and TTP, e.g. a phosphorylation site of TTP, while low-affinity ligand binding involves less attractive force. Advantageously, the method of the invention allows to screen large compound libraries and to identify the compound(s) having the best affinity to TTP. The term "binding free energy", as used herein, preferably relates to the energy released due to a bond formation or interaction between a tested compound and TTP. In one embodiment, the terms "binding energy" and "binding free energy" are used interchangeably. For example, the lower the binding free energy the tighter the binding and the higher the affinity. In one embodiment, the compound identified in step c) binds to said TTP with a binding free energy of less than -5 kcal/mol, preferably less than -7 kcal/mol, more preferably less than -8 kcal/mol. In one embodiment, said identifying in step c) comprises identifying one or more compounds binding to said TTP with a binding free energy of less than -7 kcal/mol, preferably less than -8 kcal/mol.

In one embodiment, said method, preferably said step c), comprises determining whether said compound binds to residues S60, S102, T106, S186, and/or S323 of TTP, preferably to residues S60, S186, and/or S323, wherein TTP has an amino acid sequence of SEQ ID NO. 1. In one embodiment, said method, preferably said step c), comprises determining whether said compound binds to an amino acid at position 60, 102, 106, 186, and/or 323, preferably position 60, 186, and/or 323, of an amino acid sequence of TTP, preferably of human TTP; wherein, optionally, TTP has an amino acid sequence of SEQ ID NO. 1.

In one embodiment, said binding of said compound to said TTP inhibits phosphorylation of TTP. In one embodiment, said method comprises a step d) of determining whether said compound identified in step c) inhibits phosphorylation of TTP, preferably using an in vitro assay, optionally a cell bioassay. For example, said determining in step d) may comprise treating a sample with the identified compound and comparing a level of phosphorylated TTP in said sample after said treating to a level of phosphorylated TTP in said sample prior to said treating. For example, said in vitro assay may comprise Western blotting, immunofluorescence, immunohistochemistry, immunocytochemistry, and/or ELISA. In one embodiment, said method comprises step d) of determining whether said compound identified in step c) inhibits phosphorylation of TTP, wherein said determining comprises Western blotting, immunofluorescence, immunohistochemistry, immunocytochemistry, and/or ELISA. In one embodiment, said method comprises step d) of determining whether said compound identified in step c) inhibits phosphorylation of TTP, wherein said determining comprises:
i) providing a sample comprising cells, optionally a tissue sample;
ii) treating said sample comprising cells, preferably immune cells and/or tumor cells, more preferably monocytes and/or macrophages e.g. THP-1 cells, with said compound identified in step c);
iii) optionally, stimulating said cells with a TTP phosphorylation stimulating agent, preferably with a lipopolysaccharide, IL-1, and/or PMA;
iv) determining whether said compound reduces a level of phosphorylated TTP in said treated sample of step ii), optionally said stimulated sample of step iii), compared to a control, wherein said control is a level of phosphorylated TTP determined in said sample prior to said treating in step ii), and/or is a reference value, and/or is a level of phosphorylated TTP determined in a reference sample;
wherein a reduction in the level of phosphorylated TTP indicates that said compound inhibits phosphorylation of TTP. The inventors have found that, advantageously, when the method of identifying a compound comprises determining whether said compound identified in step c) inhibits phosphorylation of TTP, compounds are identified which are even more effective in preventing or treating diseases associated with increased levels of phosphorylated TTP, such as cancer, inflammatory diseases, autoimmune diseases, and aging-associated diseases. Advantageously, by determining the inhibition of TTP phosphorylation, the compounds identified in step c) may be narrowed down to the best compound hits. In one embodiment, the expressions "inhibiting phosphorylation of TTP" and "reducing phosphorylation of TTP" are used interchangeably. In one embodiment, said step iii) of stimulating said cells with a TTP phosphorylation stimulating agent, preferably with a lipopolysaccharide, IL-1, and/or PMA, is performed prior to, simultaneously with, or after said step ii) of treating said sample comprising cells with said compound identified in step c); preferably is performed after said step ii). Advantageously, when said cells are stimulated with a phosphorylation stimulating agent, the step of determining whether said compound reduces a level of phosphorylated TTP is facilitated.

The term "sample", as used herein, relates to a specimen, preferably comprising immune cells and/or tumor cells, more preferably comprising monocytes and/or macrophages e.g. THP-1 cells. For example, the sample may be a sample of a patient, of a cell line, of a model organism, an artificial sample, and/or any other suitable sample. In one embodiment, the sample comprising cells comprises a consists of a patient sample, a sample of a model organism, and/or a sample of a cell line. In one embodiment, a sample, such as a patient sample, is any of a solid sample, such as a formalin-fixed and/or paraffin-embedded tissue, a fresh tissue, a frozen tissue, and/or a patient-derived xenograft, and a liquid sample, such as a blood sample, blood total cells, circulating cells, extracellular vesicles, exosomes, lymph fluid, saliva, body fluid, and/or tissue fluid. In a preferred embodiment, the sample comprises immune cells and/or tumor cells, more preferably comprising monocytes and/or macrophages e.g. THP-1 cells. In one embodiment, the sample is obtained from a patient, preferably noninvasively obtained from a patient. Typically, a control sample or control value is used to estimate the relative phosphorylation levels of TTP in a treated sample compared to a control.

In one embodiment, said step i) comprises providing a sample comprising cells, optionally a tissue sample, of a patient. Advantageously, when using a patient sample in step i) of a method of identifying a compound, the method allows to identify compounds which are particularly suitable for the respective patient. Particularly, by using a patient sample, it can be predicted that the identified compound has a therapeutic effect in said patient comprising reducing a level of phosphorylated TTP in said patient. Advantageously, when using a sample comprising immune cells, compounds may be identified which are effective in preventing or treating inflammatory diseases and/or immune diseases, such as autoimmune diseases, immunosenescence, and/or inflammaging. Advantageously, when using a sample comprising tumor cells, compounds may be identified which are effective in preventing or treating cancer. In one embodiment, determining whether said compound reduces a level of phosphorylated TTP comprises determining a level of phosphorylated TTP. In one embodiment, said step i) comprises providing a sample comprising cells and determining a level of phosphorylated TTP in said sample to provide a control to be used in step iv).

In one embodiment, said level of phosphorylated TTP, e.g. in step iv), is determined using an antibody or antigen-binding fragment thereof targeting phosphorylated TTP. The term "determining a level of phosphorylated TTP", as used herein, relates to assessing the level of phosphorylated TTP comprising any method capable of detecting a phosphorylation status of a protein that is known to a person skilled in the art, such as methods using reactions between an antibody (or antigen-binding fragment) and an antigen, said antigen preferably being phosphorylated TTP, for example western blotting, immunohistochemistry, immunofluorescence, mass spectrometry, flow cytometry, FACS, and ELISA. In one embodiment, the level of phosphorylated TTP is determined using an antibody targeting phosphorylated TTP and/or is determined using an antibody targeting TTP. In one embodiment, if an antibody targeting TTP is used to determine the level of phosphorylated TTP, the molecular weight and/or size difference between a phosphorylated TTP and an unphosphorylated TTP is taken into account to determine the level of phosphorylated TTP, wherein phosphorylated TTP is larger than TTP, as observed, for example, with the bands obtained in western blotting. In one embodiment, phosphorylated TTP is detected by an anti-phosphorylated TTP antibody using western blotting, immunohistochemistry, immunofluorescence, or any other method capable of detecting phosphorylated TTP known to a person skilled in the art. In one embodiment, determining a level of phosphorylated TTP relates to assessing the protein level of phosphorylated TTP and/or unphosphorylated TTP. In one embodiment, said level of phosphorylated TTP is determined using an antibody or antigen-binding fragment thereof targeting phosphorylated TTP. In one embodiment, said determining comprises detecting the total amount of phosphorylated TTP and/or detecting the fraction of phosphorylated TTP compared to total TTP. In one embodiment, an increased level of phosphorylated TTP relates to an increased total amount of phosphorylated TTP and/or to an increased phosphorylation degree of TTP, wherein an increased phosphorylation degree of TTP means that the ratio of phosphorylated TTP to unphosphorylated TTP is increased. In one embodiment, a reduced level of phosphorylated TTP relates to a reduced total amount of phosphorylated TTP and/or to a reduced phosphorylation degree of TTP, wherein a reduced phosphorylation degree of TTP means that the ratio of phosphorylated TTP to unphosphorylated TTP is reduced. The expression "reducing a level of phosphorylated TTP" relates to reducing a total amount of phosphorylated TTP and/or to reducing a phosphorylation degree of TTP, wherein a reduced phosphorylation degree of TTP means that the ratio of phosphorylated TTP to unphosphorylated TTP is reduced. In one embodiment, said reduction is a reduction by at least 15 %, preferably by at least 20 %, more preferably by at least 25 %. The inventors have found that the identified compound is highly effective in treating diseases associated with an increased level of phosphorylated TTP, if phosphorylated TTP is reduced by at least 15 %, preferably by at least 20 %, more preferably by at least 25 % by said compound.

A "control", as used herein, relates to a reference value and/or a reference sample, particularly a value and/or sample reflecting the characteristics of an untreated sample. In one embodiment, an untreated sample is a sample which has not been treated with the compound to be tested. In one embodiment, an untreated sample is a sample which has not been treated with the compound to be tested but which has been stimulated, particularly treated, with a TTP phosphorylation stimulating agent, preferably with a lipopolysaccharide, IL-1, and/or PMA. In one embodiment, the terms "reference sample" and "control sample" are used interchangeably.

In one embodiment, said stimulating said cells with a TTP phosphorylation stimulating agent, preferably with a lipopolysaccharide, IL-1, and/or PMA, in step iii), if present, comprises treating said cells with said TTP phosphorylation stimulating agent, preferably with said lipopolysaccharide, IL-1, and/or PMA. Advantageously, lipopolysaccharide, IL-1, and/or PMA typically induce(s) TTP phosphorylation. Advantageously, by stimulating said cells with said TTP phosphorylation stimulating agent, preferably with said lipopolysaccharide, IL-1, and/or PMA, TTP phosphorylation is stimulated, and the determination of a TTP phosphorylation reducing effect of said compound is facilitated. Advantageously, by stimulating TTP phosphorylation using a TTP phosphorylation stimulating agent, preferably a lipopolysaccharide, IL-1, and/or PMA, samples can be used in step i) which do not naturally comprise increased levels of phosphorylated TTP, such as samples which do not comprise cancer cells and/or senescent cells. For example, said step of stimulating said cells with a TTP phosphorylation stimulating agent, preferably with a lipopolysaccharide, IL-1, and/or PMA, may be performed if non-diseased cells are used as a sample, and said step of stimulating said cells with a TTP phosphorylation stimulating agent, such as a lipopolysaccharide, IL-1, and/or PMA, may be omitted if diseased cells having elevated levels of phosphorylated TTP, such as cancer cells, are used as a sample. In one embodiment, the TTP phosphorylation stimulating agent is a lipopolysaccharide (LPS), IL-1, phorbol 12-myristate 13-acetate (PMA), or any combination thereof, for example a combination of IL-1 and PMA or a combination of lipopolysaccharide and PMA.

The present invention also relates to a method of preventing or treating a disease in a patient having an increased level of phosphorylated TTP; wherein, preferably, said disease is selected from cancer, inflammatory diseases, autoimmune diseases, and aging-associated diseases; wherein said method comprises administering a therapeutically effective amount of a compound identified using a method of identifying a compound which binds to tristetraprolin (TTP), as defined herein, to a patient in need thereof. Advantageously, with a method of preventing or treating a disease according to the invention, diseases associated with an increased level of phosphorylated TTP, such as cancer, inflammatory diseases, autoimmune diseases, and aging-associated diseases, can be effectively treated. The embodiments and definitions described above in the context of the method of identifying a compound which binds to TTP according to the invention also apply to the method of preventing or treating a disease in a patient having an increased level of phosphorylated TTP according to the invention.

In one embodiment, the disease to be treated with a method of preventing or treating of the invention is a disease associated with an increased level of phosphorylated TTP. In one embodiment, the method of preventing or treating a disease according to the invention is a method of preventing or treating a disease associated with an increased level of phosphorylated TTP. In one embodiment, the patient is characterized by having cells with an increased level of phosphorylated TTP, such as cancer cells or senescent cells. In one embodiment, the disease is a disease associated with an increased level of phosphorylated TTP, preferably selected from cancer, inflammatory diseases, autoimmune diseases, and aging-associated diseases. In one embodiment, aging-associated diseases are selected from cardiovascular diseases such as atherosclerosis and artery narrowing e.g. artery stenosis, type-II diabetes, arthritis, Alzheimer's disease, Parkinson's disease, chronic obstructive pulmonary disease, and stroke.

The term "patient", as used herein, refers to a human or an animal, particularly having cells which are characterized by an increased level of phosphorylated TTP. In one embodiment, the patient is characterized by increased levels of phosphorylated TTP, particularly increased levels of phosphorylated TTP in said patient compared to a healthy individual and/or increased levels of phosphorylated TTP in diseased cells of said patient compared to non-diseased cells. The terms "subject" and "individual", as used herein, are used synonymously, and preferably relate to a human or an animal.

The terms "increased TTP phosphorylation", "increased phosphorylation", and "increased level of phosphorylated TTP", as used herein, refer to an elevated phosphorylation level of TTP in a patient and/or sample, particularly a patient sample, compared to the phosphorylation level of TTP in a control, particularly a non-diseased control such as a healthy individual, optionally referred to as "normal phosphorylation". An elevated phosphorylation level may also be referred to as "increased phosphorylation level". In one embodiment, an increased phosphorylation is an at least 5 % increased phosphorylation level, preferably at least 15 % increased phosphorylation level in a sample compared to a control, particularly in a patient compared to a healthy individual. The terms "decreasing phosphorylation" or "reducing phosphorylation", as used herein, relate to decreasing elevated phosphorylation levels of TTP, to normalize said increased phosphorylation to normal phosphorylation. In one embodiment, said decreasing phosphorylation comprises a decrease by at least 15 %, preferably by at least 20 %, more preferably by at least 25 %. Methods for determining the phosphorylation level of a protein such as TTP are known to a person skilled in the art, and include western blot, ELISA, microarrays, immunohistochemistry, immunofluorescence, and mass spectrometry.

In one embodiment, a control is a sample and/or value indicating normal phosphorylation. The term "normal phosphorylation" or "normal phosphorylation levels", as used herein, refers to phosphorylation levels in non-diseased cells, non-diseased samples, and/or healthy individuals. In one embodiment, normal phosphorylation relates to phosphorylation levels of TTP in non-diseased cells. In one embodiment, the term "non-diseased sample" relates to a sample which does not comprise diseased cells. In one embodiment, the terms "non-diseased subject" and "non-diseased individual" relate to a subject and individual, respectively, which substantially do not comprise diseased cells, tissues, or organs. The terms "normalizing" and "normalizing phosphorylation", as used herein, relate to normalizing or restoring phosphorylation levels of TTP to non-diseased phosphorylation levels, which can be achieved by administering a therapeutically effective amount of a compound identified using a method of identifying of the invention to a patient in need thereof having increased TTP phosphorylation levels. In one embodiment, a "normalizing effect" refers to an effect, preferably an effect of a compound, which induces a normalization of increased TTP phosphorylation levels in diseased cells towards the TTP phosphorylation levels typically found in non-diseased cells.

The term "administering", as used herein, refers to applying a compound, such as a compound identified using a method of identifying of the invention, to a target, such as a patient and/or a sample of a patient. In one embodiment, administering relates to *in vitro* and/or in *vivo* administration. In one embodiment, administering relates to intravenous, oral, nasal, mucosal, intrabronchial, intrapulmonary, intradermal, subcutaneous, intramuscular, intravascular, intrathecal, intraocular, intraarticular, intranodal, intratumoral, or intrametastatical administration of a compound to a patient in need thereof. In one embodiment, administering may also relate to *in vitro* administration, namely to incubating a cell and/or tissue, e.g. a sample obtained from a patient, with a compound.

In one embodiment, the method of preventing or treating a disease comprises administering a therapeutically effective amount of a compound identified using a method of identifying a compound, wherein said method of identifying a compound comprises step d) of determining whether said compound identified in step c) inhibits phosphorylation of TTP, comprising, in step i), providing a sample of the patient to be treated with the method of preventing or treating a disease of the invention.

The term "therapeutically effective amount", as used herein, refers to an amount of a drug, such as an amount of a compound identified using a method of identifying of the invention, which is in the range between the dose sufficient to evoke a therapeutic effect and the maximum tolerated dose. In one embodiment, a method of preventing or treating a disease according to the present invention comprises administering a therapeutically effective amount of the compound to a patient in need thereof having an increased level of phosphorylated TTP compared to a control. In one embodiment, said therapeutically effective amount is in a dose range established for a different method of treatment comprising administering said compound, wherein said different method of treatment is for a disease which is not characterized by increased TTP phosphorylation levels in diseased cells compared to non-diseased cells. In one embodiment, the compound is selected from acetyldigitoxin, digitoxin, ergotamine, bromocriptine, dihydroergotoxine, dihydroergotamine, ergoloid mesylate, conivaptan, teniposide, tubocurarin, differin, dutasteride, itraconazole, paclitaxel, lurasidone, novobiocin, praziquantel, loperamide, pranlukast, fluspirilene, antrafenine, desloratadin, dihydroergotamine, ergotamine, glimepiride, glipizide, darifenacin, tadalafil, conivaptan, danazol, dutasteride, tasosartan, metocurine, nilotinib, telithromycin, irinotecan, 5-bromo-2-chloro-N-[3-methyl-4-(2-oxo-2H-chromen-3-yl)phenyl]benzamide, maraviroc, and saquinavir; wherein, preferably, the compound is selected from glipizide, loperamide, praziquantel, glimepiride, fluspirilene, desloratadin, ergotamine, and teniposide.

The present invention also relates to a compound for use in a method of preventing or treating a disease in a patient having an increased level of phosphorylated TTP; wherein, preferably, said disease is selected from cancer, inflammatory diseases, autoimmune diseases, and aging-associated diseases; wherein said method comprises administering a therapeutically effective amount of a compound identified using a method of identifying a compound which binds to tristetraprolin (TTP), as defined herein, to a patient in need thereof. In a preferred embodiment, said compound for use is a compound identified using a method of identifying a compound which binds to TTP according to the invention. In a preferred embodiment, said compound for use is for use in a method of preventing or treating a disease in a patient having an increased level of phosphorylated TTP according to the invention. Advantageously, with a compound for use of the invention, diseases associated with an increased level of phosphorylated TTP, such as cancer, inflammatory diseases, autoimmune diseases, or aging-associated diseases, can be effectively treated. The embodiments and definitions described above in the context of the method of identifying a compound which binds to TTP and/or in the context of a method of preventing or treating a disease also apply to the compound for use in a method of preventing or treating a disease according to the invention. In a preferred embodiment, the compound for use of the invention is for use in a method of preventing or treating a disease in a patient having an increased level of phosphorylated TTP as defined herein.

In one embodiment, the compound has a therapeutic effect by reducing phosphorylation of TTP. In one embodiment, the compound is selected from acetyldigitoxin, digitoxin, ergotamine, bromocriptine, dihydroergotoxine, dihydroergotamine, ergoloid mesylate, conivaptan, teniposide, tubocurarin, differin, dutasteride, itraconazole, paclitaxel, lurasidone, novobiocin, praziquantel, loperamide, pranlukast, fluspirilene, antrafenine, desloratadin, dihydroergotamine, ergotamine, glimepiride, glipizide, darifenacin, tadalafil, conivaptan, danazol, dutasteride, tasosartan, metocurine, nilotinib, telithromycin, irinotecan, 5-bromo-2-chloro-N-[3-methyl-4-(2-oxo-2H-chromen-3-yl)phenyl]benzamide, maraviroc, and saquinavir; wherein, preferably, the compound is selected from glipizide, loperamide, praziquantel, glimepiride, fluspirilene, desloratadin, ergotamine, and teniposide.

The term "AU-rich element" or "ARE", as used herein, refers to an adenylate-uridylate-rich element in the 3' untranslated region of a mRNA. AREs are a determinant of RNA stability, and often occur in mRNAs of proto-oncogenes, nuclear transcription factors, and cytokines. TTP is an ARE-binding protein (ARE-BP) which binds to AREs and destabilizes the mRNA. AU-rich mRNAs may code for proteins involved in inflammation, immune response, cell cycle, apoptosis, signaling, and matrix modeling. A key RNA-binding protein that promotes AU-rich mRNA deadenylation and decay is the zinc finger protein, tristetraprolin (TTP/ZFP36). The term "TTP" or "tristetraprolin", as used herein, refers to a protein which binds to AU-rich elements (AREs) in the 3'-untranslated regions of ARE-containing mRNAs, and promotes degradation of said mRNAs. TTP is also known as zinc finger protein 36 homolog (ZFP36). In one embodiment, interactions of TTP and target mRNAs are affected by the phosphorylation state of TTP. For example, phosphorylated TTP may be unable to promote ARE-mRNA decay, and thus the abundance of proteins involved in inflammation, cancer, and aging is increased and the half-life of these proteins is prolonged.

The terms "of the [present] invention", "in accordance with the invention", "according to the invention" and the like, as used herein are intended to refer to all aspects and embodiments of the invention described and/or claimed herein. As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

### BRIEF DESCRIPTION OF THE FIGURES

The present invention is now further described by reference to the following figures.

All methods mentioned in the figure descriptions below were carried out as described in detail in the examples.

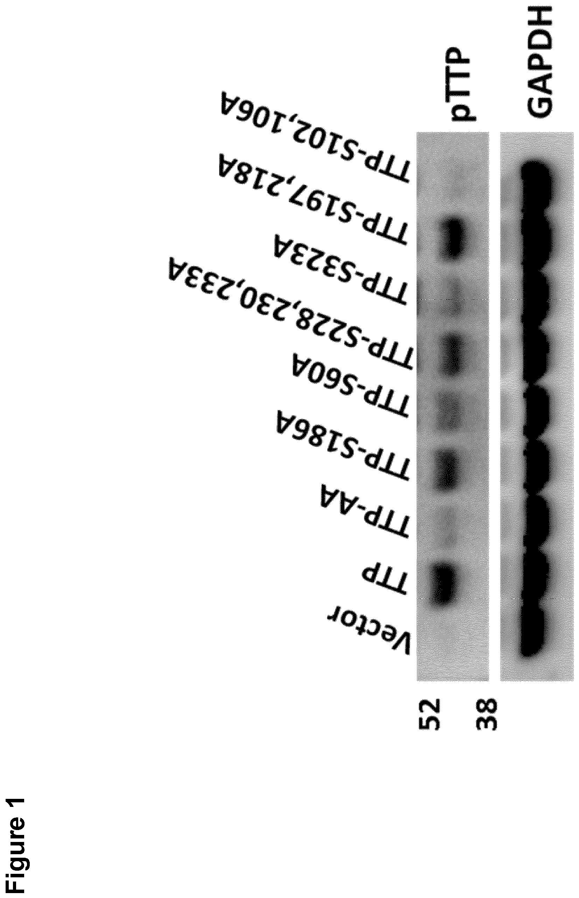

**Figure 1** shows a western blot demonstrating that mutations of S60, S186, S323, S102/S106, and S60/S186 (S60/S186 is labelled as "TTP-AA") result in a reduced abundance of phosphorylated TTP. TTP mutants were generated by site-directed mutagenesis and cloning in expression vectors. HEK293 were transfected with plasmids expressing either wild-type TTP or TTP mutants. The indicated mutations refer to the position of the mutated amino acid serine and serine-to-alanine mutations.

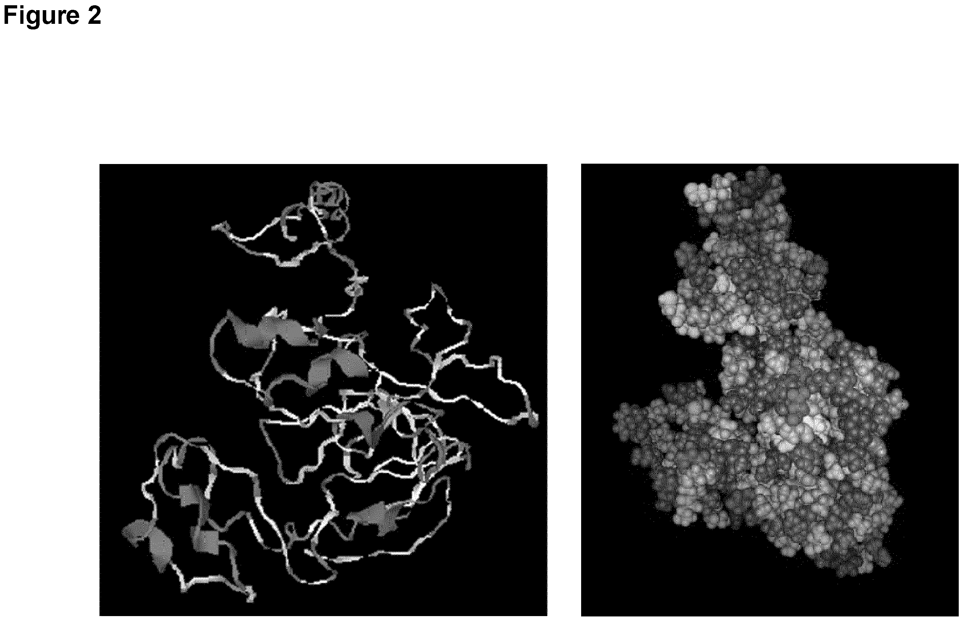

**Figure 2** shows the predicted structure of tristetraprolin protein (left) with space fill (surface) format (right). I-Tasser was used to predict the structure of human ZFP36 protein (tristetraprolin, TTP). The best fit model output of I-Tasser predicted protein structures are shown.

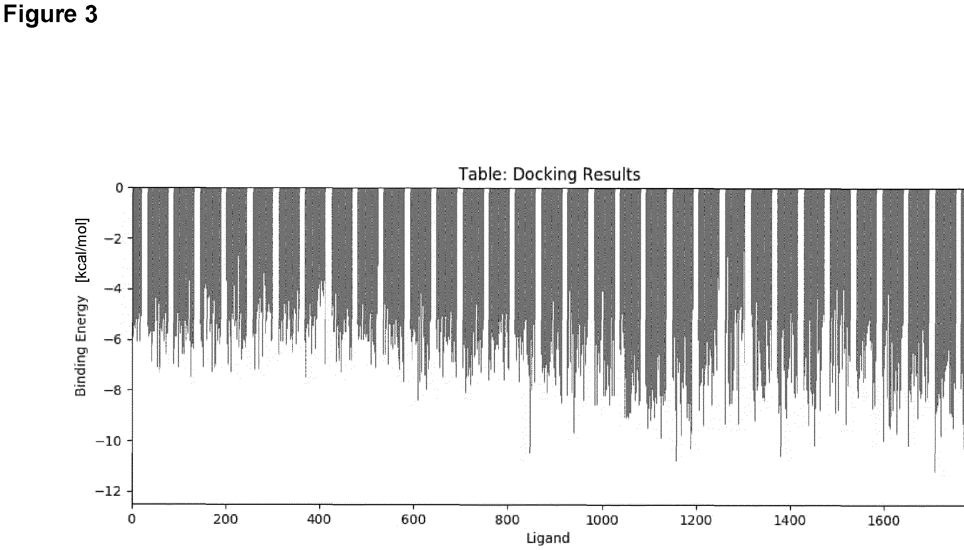

**Figure 3** shows a graphical representation of the docking comprising using binding free energies.
**Figure 4** shows that the predicted compounds indeed show high binding affinities. It is shown that the exemplary compounds have an effect on the reduction of phosphorylated TTP, as predicted by molecular docking virtual screening of a library of 1500 FDA approved drugs.
THP-1 monocytic cell line was pre-treated for 6 h, and then stimulated with LPS for 2 h in the presence or absence of the indicated drug (1 µM).
**Figure 5** shows that the predicted compounds indeed show high binding affinities. It is shown that the exemplary compounds have an effect on the reduction of phosphorylated TTP, as predicted by molecular docking virtual screening of a library of 1500 FDA approved drugs.
THP-1 monocytic cell line was pre-treated with the indicated drugs, and then stimulated with LPS for the indicated time in the presence or absence of the indicated drug (1 µM).

In the following, reference is made to the examples, which are given to illustrate, not to limit the present invention.

### EXAMPLES

### Example 1:

FDA-approved compounds were obtained as .sdf files from Zinc database and were used as input for Pyrx virtual screening program utilizing AutoVina docking program. The compounds were pre-processed by pyrx and docked with the TTP/ZPF36 3D structure obtained from I-Tasser prediction program. Selected top compounds from the molecular-docking based virtual screening with the lowest minimal binding free energies (which correspond to the best binding affinities of the compound-protein interaction) were tested for their effect of the abundance of phosphorylated TTP.

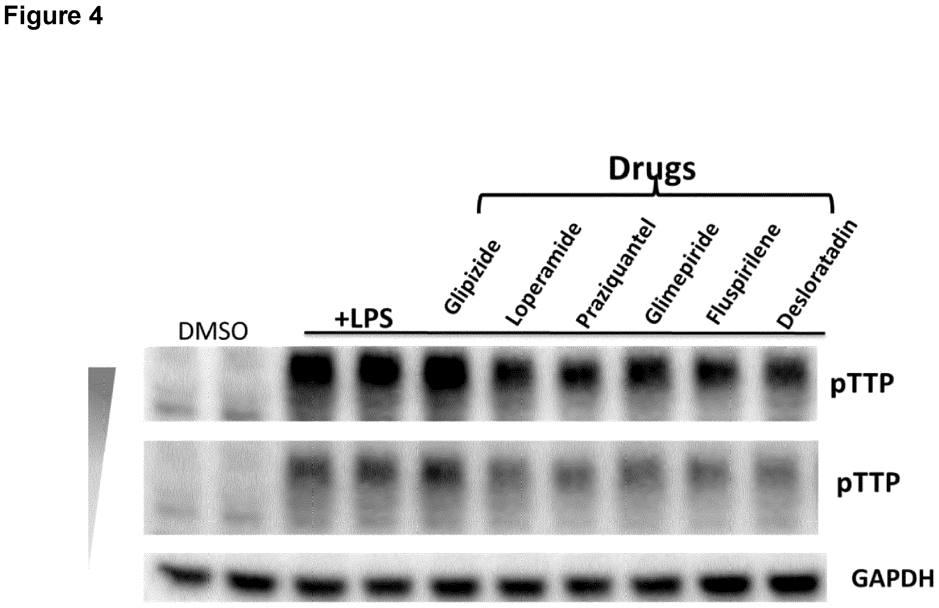

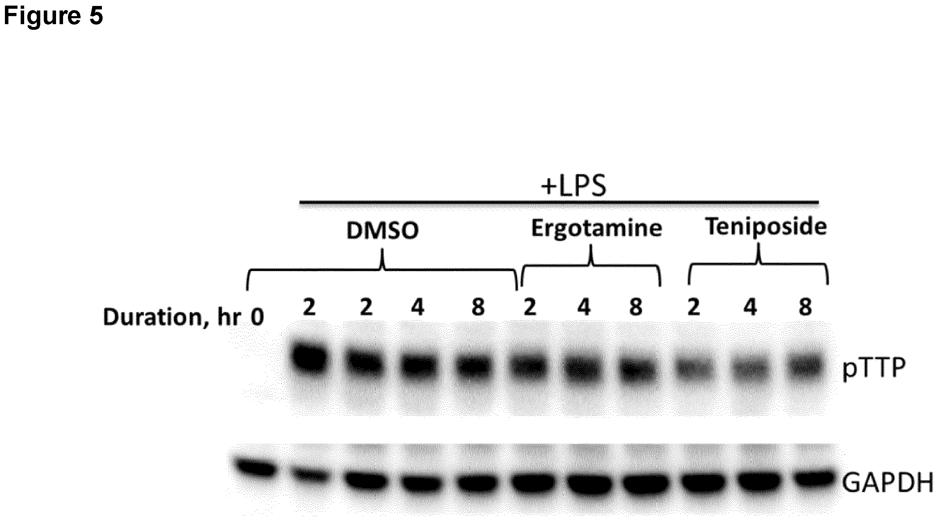

THP-1 monocytic cell line was obtained from ATCC and cultured in complete RPMI medium with 10% FBS. The cells were seeded 1 million cells per well in six-well plates and treated with DMSO as a control vehicle or compound (1 µM) for 1 hr followed by treatment with LPS 1 µg/ml as inducer of TTP phosphorylation pathway for the indicated periods of time (Fig. 4 and 5). Cells were directly lysed in NP 40 lysis buffer. Lysates were sonicated to shear DNA, and equivalent lysate levels were loaded onto SDS PAGE gel, blotted to nitrocellulose membrane, and probed with antibodies to TTP. The affinity-purified TTP rabbit polyclonal antibody was custom-made with Genescript against the C-terminal end of TTP and was used previously to detect total TTP which is mostly phosphorylated by LPS treatment.

As demonstrated, the method of the invention successfully identified compounds which reduce TTP phosphorylation.

### Example 2:

Ligand-protein binding affinities of compounds identified using a method of the invention are listed in the following tables. The method of the invention efficiently identified compounds binding to TTP with high affinity. Furthermore, as shown in tables 2 and 3, the method of the invention efficiently identified compounds binding to TTP phosphorylation sites.

**Table 1: Top Ligands binding to ZFP36/TTP**

| Ligand | Compound | Target | Binding Energy [kcal/mol] |
|---|---|---|---|
| zinc96006012 | Acetyldigitoxin | ttp_model | -11.9 |
| zinc95862733 | Digitoxin | ttp_model | -11.7 |
| zinc52955754 | Ergotamine | ttp_model | -11.2 |
| zinc53683151 | Bromocriptine | ttp_model | -10.9 |
| zinc14880002 | Dihydroergotoxine | ttp_model | -10.9 |
| zinc03978005 | Dihydroergotamine | ttp_model | -10.8 |
| zinc33359785 | Ergoloid Mesylate | ttp_model | -10.8 |
| zinc12503187 | Conivaptan | ttp_model | -10.6 |
| zinc04099009 | Teniposide | ttp_model | -10.6 |
| zinc03978083 | Tubocurarin | ttp_model | -10.6 |
| zinc03784182 | Differin | ttp_model | -10.5 |
| zinc03932831 | Dutasteride | ttp_model | -10.5 |
| zinc04097343 | Itraconazole | ttp_model | -10.3 |
| zinc96006020 | Paclitaxel | ttp_model | -10.3 |
| zinc33974796 | Lurasidone | ttp_model | -10.2 |
| zinc14879999 | Novobiocin | ttp_model | -10.2 |
| ZINC00000655 | Praziquantel | ttp_model | -8.1 |
| zinc00537928 | Loperamide | ttp_model | -7.2 |

**Table 2: Ligand compounds affinity to grid around serine 186 ZFP36/TTP**

| Ligand | Compound | Target | Binding Energy [kcal/mol] |
|---|---|---|---|
| zinc26683062 | Pranlukast | ttp_m_186 | -9.0 |
| ZINC00537755 | Fluspirilene | ttp_m_186 | -8.6 |
| zinc53073961 | Antrafenine | ttp_m_186 | -8.5 |
| ZINC00001261 | Desloratadin | ttp_m_186 | -8.5 |
| zinc03978005 | Dihydroergotamine | ttp_m_186 | -8.4 |
| zinc52955754 | Ergotamine | ttp_m_186 | -8.2 |
| ZINC00537791 | Glimepiride | ttp_m_186 | -8.3 |
| ZINC00537791 | Glipizide | ttp_m_186 | -8.3 |
| zinc33359785 | Darifenacin | ttp_m_186 | -8 |
| zinc03993855 | Tadalafil | ttp_m_186 | -8 |
| zinc12503187 | Conivaptan | ttp_m_186 | -7.9 |
| zinc03881958 | Danazol | ttp_m_186 | -7.9 |
| zinc03932831 | Dutasteride | ttp_m_186 | -7.9 |
| zinc13444037 | Tasosartan | ttp_m_186 | -7.9 |

**Table 3: Ligand compounds affinity to grid around serine 323 ZFP36/TTP**

| Ligand | Compound | Target | Binding Energy [kcal/mol] |
|---|---|---|---|
| zinc33359785 | Dihydroergotoxin Mesilat | ttp_m_323 | -10.7 |
| zinc14880002 | Dihydroergotoxine | ttp_m_323 | -10.5 |
| zinc03978005 | Dihydroergotamine Mesylate | ttp_m_323 | -10.4 |
| zinc04097448 | Metocurine | ttp_m_323 | -10.4 |
| zinc06716957 | Nilotinib | ttp_m_323 | -10.2 |
| zinc33974796 | Lurasidone | ttp_m_323 | -10.1 |
| zinc95617679 | Telithromycin | ttp_m_323 | -10.1 |
| zinc01612996 | Irinotecan | ttp_m_323 | -10 |
| zinc52955754 | Ergotamine | ttp_m_323 | -10 |
| zinc53073961 | Antrafenine | ttp_m_323 | -10 |
| zinc12503187 | Conivaptan Hydrochloride | ttp_m_323 | -9.9 |
| zinc03932831 | Dutasteride | ttp_m_323 | -9.8 |
| zinc96006012 | 5-bromo-2-chloro-N-[3-methyl-4-(2-oxo-2H-chromen-3-yl)phenyl]benzamide | ttp_m_323 | -9.8 |
| zinc26683062 | Pranlukast | ttp_m_323 | -9.7 |
| zinc03817234 | Maraviroc | ttp_m_323 | -9.6 |
| zinc04099009 | Teniposide | ttp_m_323 | -9.6 |
| zinc26985532 | Saquinavir | ttp_m_323 | -9.6 |
| zinc53683151 | Bromocriptine | ttp_m_323 | -9.6 |
| zinc95862733 | Digitoxin | ttp_m_323 | -9.6 |
| zinc04099008 | Teniposide | ttp_m_323 | -9.5 |

As shown in Fig. 4 and Fig. 5, the identified compounds effectively reduce TTP phosphorylation. Accordingly, the method of the invention is a highly efficient method for identifying compounds that may be used in the prevention and treatment of diseases that are characterized by aberrant TTP phosphorylation, particularly characterized by increased levels of phosphorylated TTP, such as cancer, inflammatory diseases, autoimmune diseases, and aging-associated diseases.

### REFERENCES

[1] Brook, M., C. R. Tchen, T. Santalucia, J. McIlrath, J. S. Arthur, J. Saklatvala and A. R. Clark (2006). "Posttranslational regulation of tristetraprolin subcellular localization and protein stability by p38 mitogen-activated protein kinase and extracellular signal-regulated kinase pathways." Mol Cell Biol 26(6):2408-2418.
[2] Hitti, E., T. Iakovleva, M. Brook, S. Deppenmeier, A. D. Gruber, D. Radzioch, A. R. Clark, P. J. Blackshear, A. Kotlyarov and M. Gaestel (2006). "Mitogen-activated protein kinase-activated protein kinase 2 regulates tumor necrosis factor mRNA stability and translation mainly by altering tristetraprolin expression, stability, and binding to adenine/uridine-rich element." Mol Cell Biol 26(6): 2399-2407.
[3] Vlasova-St Louis, I. and P. R. Bohjanen (2016). "Feedback Regulation of Kinase Signaling Pathways by AREs and GREs." Cells 5(1): 4.
[4] Cao H, Lin R (2008). "Phosphorylation of recombinant tristetraprolin in vitro." Protein J. 27:163-9.

The features of the present invention disclosed in the specification, the claims, and/or in the accompanying figures may, both separately and in any combination thereof, be material for realizing the invention in various forms thereof.

## Claims

1. A method of identifying a compound which binds to tristetraprolin (TTP), preferably a compound which inhibits phosphorylation of TTP, comprising the following steps:
a) providing a three-dimensional structure of TTP; wherein, preferably, said providing comprises predicting a three-dimensional structure of TTP using a computer-implemented method;
b) preferably, identifying a phosphorylation site of TTP; wherein, preferably, said phosphorylation site of TTP comprises or consists of serine and/or threonine, preferably serine;
c) identifying a compound which binds to said three-dimensional structure of TTP provided in step a), preferably a compound which binds to said phosphorylation site of TTP identified in step b), if present; wherein, optionally, said identifying comprises virtually screening a compound library comprising at least one compound;
d) optionally, determining whether said compound identified in step c) inhibits phosphorylation of TTP, preferably using an in vitro assay, optionally a cell bioassay.

2. The method according to claim 1, wherein said providing in step a) comprises predicting a three-dimensional structure of TTP using a computer-implemented method comprising fold recognition;
wherein, preferably, said predicting comprises secondary structure prediction, template detection, fragment structure assembly, model selection, atomic-level structure refinement, and/or structure-based biology function annotation;
wherein, more preferably, said predicting comprises iterative threading assembly refinement.

3. The method according to claim 1 or 2, wherein said method comprises identifying a phosphorylation site of TTP, wherein said identifying a phosphorylation site comprises predicting a phosphorylation site using a computer-implemented method and/or comprises site-directed mutagenesis.

4. The method according to any one of the foregoing claims, wherein said identifying a compound in step c) comprises identifying a compound which binds to said three-dimensional structure of TTP provided in step a), preferably a compound which binds to said phosphorylation site of TTP identified in step b), if present; wherein said identifying comprises screening, preferably virtually screening, a compound library comprising at least one compound, preferably a plurality of compounds; wherein, optionally, said at least one compound, preferably said plurality of compounds, is/are selected from small molecule inhibitors, antibodies, and antigen-binding fragments thereof, preferably from protein kinase inhibitors.

5. The method according to any one of the foregoing claims, wherein said identifying a compound in step c) comprises virtually screening a compound library comprising at least one compound by molecular docking of said at least one compound to TTP, preferably comprising determining whether said at least one compound binds to TTP;
wherein, preferably, said identifying a compound in step c) comprises virtually screening a compound library comprising at least one compound by molecular docking of said at least one compound to said phosphorylation site of TTP identified in step b), if present, preferably comprising determining whether said at least one compound binds to TTP, more preferably comprising determining whether said at least one compound inhibits phosphorylation of TTP.

6. The method according to any one of the foregoing claims, wherein said identifying a compound in step c) comprises virtually screening a compound library comprising at least one compound by molecular docking of said at least one compound to TTP, preferably to said phosphorylation site of TTP identified in step b), if present, wherein said virtually screening comprises docking of said at least one compound to a set of grids describing the three-dimensional structure of TTP provided in step a), preferably describing said phosphorylation site of TTP identified in step b), if present.

7. The method according to any one of the foregoing claims, wherein said TTP in step a) is human TTP, preferably TTP having an amino acid sequence of SEQ ID NO. 1.

8. The method according to any one of the foregoing claims, wherein said method, optionally said step c), comprises determining whether said compound binds to residues S60, S102, T106, S186, and/or S323 of TTP, preferably to residues S60, S186, and/or S323, wherein TTP has an amino acid sequence of SEQ ID NO. 1.

9. The method according to any one of the foregoing claims, wherein the compound identified in step c) binds to said TTP with a binding free energy of less than -5 kcal/mol, preferably less than -7 kcal/mol, more preferably less than -8 kcal/mol.

10. The method according to any one of the foregoing claims, wherein said identifying in step c) comprises virtually screening a compound library comprising at least 5 compounds, preferably at least 10 compounds, more preferably at least 100 compounds, even more preferably at least 500 compounds, even more preferably at least 1000 compounds such as at least 1400 compounds; wherein, optionally, said compounds are selected from small molecule inhibitors, antibodies, and antigen-binding fragments thereof, preferably from protein kinase inhibitors;
wherein, optionally, said identifying comprises identifying one or more compounds binding to said TTP with a binding free energy of less than -7 kcal/mol, preferably less than -8 kcal/mol, and/or identifying one or more compounds having a lower binding free energy than 80%, preferably than 90% of the compounds of the compound library.

11. The method according to any one of the foregoing claims, wherein said method comprises step d) of determining whether said compound identified in step c) inhibits phosphorylation of TTP, wherein said determining comprises:
i) providing a sample comprising cells, optionally a tissue sample;
ii) treating said sample comprising cells, preferably immune cells and/or tumor cells, more preferably monocytes and/or macrophages e.g. THP-1 cells, with said compound identified in step c);
iii) optionally, stimulating said cells with a TTP phosphorylation stimulating agent, preferably with a lipopolysaccharide, IL-1, and/or PMA;
iv) determining whether said compound reduces a level of phosphorylated TTP in said treated sample compared to a control, wherein said control is a level of phosphorylated TTP determined in said sample prior to said treating in step ii), and/or is a reference value, and/or is a level of phosphorylated TTP determined in a reference sample;
wherein a reduction in the level of phosphorylated TTP indicates that said compound inhibits phosphorylation of TTP.

12. The method according to claim 11, wherein said reduction is a reduction by at least 15 %, preferably by at least 20 %, more preferably by at least 25 %; and/or
wherein said level of phosphorylated TTP is determined using an antibody or antigen-binding fragment thereof targeting phosphorylated TTP.

13. The method according to any one of the foregoing claims, wherein said method comprises step d) of determining whether said compound identified in step c) inhibits phosphorylation of 1TP, wherein said determining comprises Western blotting, immunofluorescence, immunohistochemistry, immunocytochemistry, and/or ELISA.

14. A compound for use in a method of preventing or treating a disease in a patient having an increased level of phosphorylated TTP; wherein, preferably, said disease is selected from cancer, inflammatory diseases, autoimmune diseases, and aging-associated diseases; wherein said method comprises administering a therapeutically effective amount of a compound identified using a method according to any one of claims 1-13 to a patient in need thereof.

15. The compound for use according to claim 14, wherein the compound is selected from acetyldigitoxin, digitoxin, ergotamine, bromocriptine, dihydroergotoxine, dihydroergotamine, ergoloid mesylate, conivaptan, teniposide, tubocurarin, differin, dutasteride, itraconazole, paclitaxel, lurasidone, novobiocin, praziquantel, loperamide, pranlukast, fluspirilene, antrafenine, desloratadin, dihydroergotamine, ergotamine, glimepiride, glipizide, darifenacin, tadalafil, conivaptan, danazol, dutasteride, tasosartan, metocurine, nilotinib, telithromycin, irinotecan, 5-bromo-2-chloro-N-[3-methyl-4-(2-oxo-2H-chromen-3-yl)phenyl]benzamide, maraviroc, and saquinavir;
wherein, preferably, the compound is selected from glipizide, loperamide, praziquantel, glimepiride, fluspirilene, desloratadin, ergotamine, and teniposide.
